# EUROPEAN PATENT SPECIFICATION

(11) **EP 4 043 551 B1**
(45) Date of publication and mention of the grant of the patent: **01.01.2025**
(21) Application number: 21156945.4
(22) Date of filing: 12.02.2021
(51) Int. Cl.: C12N 5/00, A23J 3/18, A23J 3/32

(54) **NUTRIENT MEDIA FOR CELL CULTURE CONTAINING PLANT PROTEIN HYDROLYSATES**
NÄHRMEDIEN MIT PFLANZENPROTEINHYDROLYSATEN FÜR ZELLKULTUR
MILIEUX NUTRITIFS POUR CULTURE CELLULAIRE CONTENANT DES HYDROLYSATS DE PROTÉINES VÉGÉTALES

(43) Date of publication of application: 17.08.2022
(73) Proprietor: Bühler AG, 9240 Uzwil (CH)
(72) Inventor: CORDESMEYER, Frank, 48429 Rheine (DE); O'NIEN, Jay, 9000 St. Gallen (CH); CONDE-PETIT, Beatrice, 8048 Zürich (CH)
(74) Representative: Leimgruber, Fabian Alfred Rupert

(56) References cited:
- JP-A- 2013 247 927
- US-A1- 2006 286 668
- JARUNRATTANASRI ARPORN ET AL: "Aroma Components of Acid-Hydrolyzed Vegetable Protein Made by Partial Hydrolysis of Rice Bran Protein", JOURNAL OF AGRICULTURAL AND FOOD CHEMISTRY, vol. 55, no. 8, 1 April 2007 (2007-04-01), US, pages 3044 - 3050, XP055823220, ISSN: 0021-8561, Retrieved from the Internet <URL:https://pubs.acs.org/doi/pdf/10.1021/jf0631474> DOI: 10.1021/jf0631474
- HANSAWASDI CHANIDA ET AL: "Potential Prebiotic Oligosaccharide Mixtures from Acidic Hydrolysis of Rice Bran and Cassava Pulp", PLANTS FOODS FOR HUMAN NUTRITION, KLUWER ACADEMIC PUBLISHERS, NL, vol. 72, no. 4, 3 October 2017 (2017-10-03), pages 396 - 403, XP036377921, ISSN: 0921-9668, [retrieved on 20171003], DOI: 10.1007/S11130-017-0636-Z
- SATOSHI TERADA ET AL: "Culture supplement extracted from rice bran for better serum-free culture", BMC PROCEEDINGS, BIOMED CENTRAL LTD, LONDON UK, vol. 7, no. Suppl 6, 4 December 2013 (2013-12-04), pages P104, XP021170293, ISSN: 1753-6561, DOI: 10.1186/1753-6561-7-S6-P104

## Description

### FIELD OF THE INVENTION

The present invention relates to nutritional components suitable for cell culture of eukaryotic cells. These nutritional components can be prepared from plant-based protein hydrolysates. The invention further relates to nutrient media comprising these nutritional components. The nutrient media of the invention can be prepared by replacing serum components, which are common in such media, - at least partially - by the nutritional components of the invention. The nutritional components and the nutrient media of the invention are particularly suitable for use in the food industry, such as in the production of cultured meat.

### BACKGROUND OF THE INVENTION

Prior attempts have been made to use plant-based material as components for cell culture media.

WO 98/15614 A1 describes cell culture medium formulations for the *in vitro* cultivation of animal cells. These media formulations comprise at least one plant-derived peptide and/or at least one plant-derived lipid and/or at least one plant-derived fatty acid. Rice, soy, potato, and corn are named as suitable sources of proteins, peptides, lipids, and/or fatty acids. Wheat is specifically excluded as a source of plant-derived proteins. The plant peptides to be used in the formulations of WO 98/15614 A1 may be prepared by digesting plant extracts with enzymes such as trypsin or chymotrypsin. The plant peptides are to be added to the basal medium at concentrations of about 10 - 1000 mg/liter.

WO 03/045995 A2 describes the cost-effective production of recombinant glycoproteins such as human erythropoietin using a serum-free culture medium. The culture medium comprises a plant-derived peptone. The examples of WO 03/045995 A2 show a serum-free adaptation medium and a serum-free production medium. Each one of these two media contains 0.25% soya-peptone.

US 9,714,411 B2 describes animal protein-free cell culture media comprising polyamines and a plant- and/or yeast-derived hydrolysate. The total concentration of the plant- and/or yeast-derived protein hydrolysate in the cell culture medium is about 0.05% to about 5% (w/v). The plant-derived protein hydrolysate may be selected from a cereal hydrolysate and/or a soy hydrolysate. US 9,714,411 B2 defines the term "hydrolysate" as relating to any enzymatic digest of a vegetable or yeast extract.

JP2013-247927 A discloses that a rice bran extract has a cell growth promoting action, when used in cell culture medium. The rice bran extract preferably comprises 50% by mass or more of protein. The rice bran extract may be a molecular weight fractionated product, preferably in the range between 3 kDa and 10kDa. In the Examples of JP2013-247927 A, it was shown that a rice bran extract with a protein content of 55% improved proliferation of CHO cells when used in concentrations of 0.1 or 0.3 g/L, while cytotoxicity was observed at a concentration of 1 g/L.

Jarunrattanasri and co-workers studied the aroma components present in rice bran protein, especially the aroma components present in partially acid-hydrolyzed vegetable protein made by partial hydrolysis of rice bran protein (A. Jarunrattanasri et al. "Aroma Components of Acid-Hydrolyzed Vegetable Protein Made by Partial Hydrolysis of Rice Bran Protein" J. Agric. Food Chem. 2007, 55(8):3044-3050).

Hansawasdi and co-workers examined the potential prebiotic effects of oligosaccharide mixtures from acidic hydrolysis of rice bran and cassava pulp (C. Hansawasdi et al. "Potential Prebiotic Oligosaccharide Mixtures from Acidic Hydrolysis of Rice Bran and Cassava Pulp" Plants Food Hum. Nutr. 2017, 72(4):396-403).

Terada and colleagues describe a culture supplement extracted from rice bran for serum-free culture of mammalian cells (S. Terada et al. "Culture supplement extracted from rice bran for better serum-free culture" BMC Proceedings 2013, 7(Suppl 6):P104). Rice bran was extracted in an alkaline solution and then precipitated with acid.

The inventors of WO 2011/111920 A1 disclose a titration of rice hydrolysates as cell culture supplements. The rice hydrolysates were prepared by enzymatic hydrolysis.

Patent application US 2006/286668 A1 refers to a cell culture medium devoid of animal proteins and comprising at least one non-animal or plant-derived peptide. The application further refers to a cell culture medium devoid of animal proteins and comprising at least one non-animal-derived or plant-derived lipid or fatty acid.

### TECHNICAL PROBLEMS UNDERLYING THE PRESENT INVENTION

At present, nutrient media for cell culture are a mixture of about 20 to 60 high grade and pure ingredients. The high number and the high purity of the ingredients renders such nutrient media very expensive.

When using nutrient media in the food industry, especially when producing cultured meat, it is desirable to use less expensive ingredients. For ethical reasons and for safety reasons, it is also desirable to avoid ingredients from animal origin.

In the prior art, plant-based hydrolysates were used as additives for cell culture media. However, in the prior art, only small amounts of plant-based extracts were used to supplement cell culture media; e.g. only up to 1000 mg/L (= 1 g/L) in WO 98/15614 A1; about 0.25% soya-peptone (= 2.5 g/L) in WO 03/045995 A2; between 0.1 and 0.3 g/L of a protein-rich rice bran extract were used in JP2013-247927. In addition, the plant-based extracts used in the prior art were obtained from isolated plant proteins or from protein-rich plant material, which can be grouped as primary food stream material.

The present inventors have now shown for the first time that nutrient media components can be prepared using plant-based protein hydrolysates that were produced from secondary food stream material, i.e. material having lower economic value than primary food stream material.

The novel composition containing plant-based hydrolysates prepared from secondary food stream are much cheaper than the media ingredients of the prior art. These novel compositions can be used as media components and support the growth of eukaryotic cells, and can even be used in the production of cultured meat. In addition, the components and compositions of the present invention can be used for cell cultivation in an industrial scale.

This summary does not necessarily describe all advantages achieved and all problems solved by the present invention.

### SUMMARY OF THE INVENTION

In a first aspect the present invention relates to a method for the production of a plant-based hydrolysate suitable for cell culture of eukaryotic cells, said plant-based protein hydrolysate being obtained from secondary food stream material, comprising the steps:
(a) providing a plant-based material obtained from secondary food stream, wherein the plant-based material obtained from secondary food stream is obtained from rice bran, wherein said plant-based material obtained from secondary food stream has a protein content ranging from 5% to 25% (w/w dry matter) and has a fibre content ranging from 1% to 30% (w/w dry matter);
(b) adding a concentrated solution of HCl to the plant-based material, wherein the concentration of HCl is in the range from 0.2 M to 1 M, thereby preparing a mixture of acid and material;
(c) incubating the mixture of step (b) at a temperature in the range between 70°C and 90°C for a time period of between 15 hours and 36 hours, thereby preparing an acid-hydrolysate;
(d) adding an alkaline solution to the acid-hydrolysate of step (c) in an amount sufficient to neutralize the acid-hydrolysate;
(e) centrifuging the neutralized acid-hydrolysate of step (d) to precipitate insoluble material;
(f) recovering the supernatant of the centrifugation of step (e); and
(g) filtering the supernatant recovered in step (f) through a sterile filter or a combination of sterile filters, thereby obtaining the plant-based hydrolysate suitable for cell culture of eukaryotic cells.

In a second aspect the present invention relates to a plant-based hydrolysate obtainable by the method of the first aspect.

In a third aspect the present invention relates to a composition suitable for use as medium component supporting the growth of eukaryotic cells, the composition comprising:
(i) a first plant-based hydrolysate obtainable by the method of the first aspect; and
(ii) a food grade premix comprising one or more minerals, one or more vitamins, one or more amino acids, and/or one or more salts;
wherein said composition is devoid of animal proteins.

In a fourth aspect the present invention relates to a method for cultivating cells, comprising the steps.
(a) dissolving the composition according to the third aspect in a sterile aqueous solvent, thereby preparing a solution of a plant hydrolysate;
(b) adding salts, buffer, energy sources (e.g. glucose), trace minerals, essential amino acids, and/or growth factors to the solution of step (a), thereby preparing a growth medium;
(c) inoculating the growth medium of step (b) with appropriate cells, wherein the appropriate cells are selected from the group consisting of myosatellite cells, mesenchymal stromal cells, fibroblast cells, muscle cells, stem cells, and induced pluripotent stem cells (iPSCs); and
(d) culturing the inoculated growth medium of step (c) at an appropriate temperature for an appropriate time period, thereby cultivating the cells.

In a fifth aspect the present invention relates to a method for the production of the composition of the third aspect, comprising the step(s):
- mixing a plant-based hydrolysate obtainable by the method of the first aspect with a food grade premix and optionally with a plant-based hydrolysate prepared from primary food stream material by acid hydrolysis.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

As used herein, the expression material from "primary food stream" refers to plant material that is suitable for human consumption. This material is characterized by high protein content (at least 30% (w/w dry matter) but typically at least 50% (w/w dry matter)) and low fibre content (5% or less (w/w dry matter), but typically 1% or less (w/w dry matter)). Examples of primary food stream material include, without limitation, protein-rich material derived from soy, fava bean, wheat (e.g. wheat gluten), rice, pea, potato, or cotton seed.

As used herein, the expression material from "secondary food stream" refers to plant material that is generally not used for human consumption. Material from secondary food stream is mostly used as animal feed, as fertilizer or is simply burnt. As compared to material from primary food stream, secondary food stream material has a lower protein content (4% to 35%, and typically 10% to 30% (w/w dry matter)) and a higher fibre content (1% to 50%, and typically 10% to 20% (w/w dry matter)). Examples of secondary food stream material include, without limitation, rice bran, defatted rice bran, wheat bran, rye bran, maize bran, spelt bran, or brewer's spent grain.

In the context of the present invention, a "growth factor" refers to a protein or a peptide that promotes proliferation of eukaryotic cells in cell culture. Examples of growth factors usable in the present invention include, without limitation insulin and insulin-like growth factors (IGF).

As used herein, the term "about" refers to numerical values ranging from 5% below the indicated numerical value to 5% above the indicated numerical value. For example, a protein content of *"about 10% (w*/*w dry matter) "* encompasses a protein content ranging from 9.5% (w/w dry matter) to about 10.5% (w/w dry matter). As a further example, a temperature of *"about 80°C"* refers to a temperature range between 76°C and 84°C.

### Embodiments of the Invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a third aspect the present invention is directed to a composition comprising:
(i) a first plant-based hydrolysate obtainable by the method of the first aspect; and
(ii) a food grade premix comprising one or more minerals, one or more vitamins, one or more amino acids, and/or one or more salts;
wherein said composition is devoid of animal proteins.

In a preferred embodiment of the third aspect, the secondary food stream material has a protein content ranging from 6% to 25%, preferably from 7% to 25%, preferably from 8% to 25%, preferably from 9% to 25%, preferably from 10% to 25%, preferably from 11% to 25%, preferably from 12% to 25%, preferably from 13% to 25%, preferably from 14% to 25%, and most preferably from 15% to 25% (w/w dry matter).

In a preferred embodiment of the third aspect, the secondary food stream material has a fibre content ranging from 2% to 30%, preferably from 3% to 30%, preferably from 4% to 30%, preferably from 5% to 30%, and most preferably from 6% to 25% (w/w dry matter).

In all embodiments of the third aspect, the secondary food stream material is or is prepared from rice bran.

Further disclosed herein but not claimed are compositions, in which the secondary food stream material is or is prepared from wheat bran, rye bran, maize bran, spelt bran, or brewer's spent grain.

In a preferred embodiment of the third aspect, the secondary food stream material is or is prepared from
- rice bran having a protein content in the range from 5% to 20% (w/w dry matter) and a fibre content in the range from 2% to 30% (w/w dry matter).

Further disclosed herein but not claimed are compositions, in which the secondary food stream material is or is prepared from
- wheat bran having a protein content in the range from about 12% to about 24% (w/w dry matter) and a fibre content in the range from about 5% to about 15% (w/w dry matter); or
- rye bran having a protein content in the range from about 12% to about 20% (w/w dry matter) and a fibre content in the range from about 4% to about 30% (w/w dry matter); or
- maize bran having a protein content in the range from about 5% to about 20% (w/w dry matter) and a fibre content in the range from about 4% to about 25% (w/w dry matter); or
- spelt bran having a protein content in the range from about 12% to about 24% (w/w dry matter) and a fibre content in the range from about 5% to about 16% (w/w dry matter); or
- brewer's spent grain having a protein content in the range from about 16% to about 35% (w/w dry matter) and a fibre content in the range from about 10% to about 25% (w/w dry matter).

In a preferred embodiment of the third aspect, the first plant-based hydrolysate is present in the composition in a concentration in the range from 0.1% to 50% (w/w).

In a preferred embodiment of the third aspect,
- the one or more minerals in the food grade pre-mix are selected from the group consisting of Mg²⁺, Ca²⁺, Fe²⁺, Fe³⁺, Cu²⁺, Zn²⁺, and Mn²⁺;
- the one or more vitamins in the food grade pre-mix are selected from the group consisting of ascorbic acid, biotin, choline, D-calcium pantothenate, folic acid, niacinamide, pyridoxine, pyridoxal, riboflavin, thiamine, vitamin B12, and i-inositol;
- the one or more amino acids in the food grade pre-mix are selected from the group consisting of glycine, L-alanine, L-arginine, L-asparagine, L-aspartic acid, L-cysteine, L-cystine, L-glutamic acid, L-glutamine, L-histidine, L- isoleucine, L-leucine, L-lysine, L-methionine, L-phenylalanine, L-proline, L-serine, L-threonine, L-tryptophan, L-tyrosine, and L-valine; and/or
- the one or more salts in the food grade pre-mix are selected from the group consisting of NaCl, KCl, Na₂HPO₄, NaH₂PO₄, and NaHCO₃.

In a preferred embodiment of the third aspect, the food grade premix is present in the composition in a concentration in the range from 10% to 99.9% (w/w).

In a preferred embodiment of the third aspect, the composition further comprises:
(iii) a second plant-based hydrolysate prepared from primary food stream material by acid hydrolysis, wherein said primary food stream material has a protein content of at least 30% (w/w dry matter) protein and a fibre content of 5% (w/w dry matter) or less.

In a preferred embodiment of the third aspect, the primary food stream material has a protein content of at least 32%, preferably at least 34%, preferably at least 36%, preferably at least 38%, preferably at least 40%, preferably at least 42%, preferably at least 44%, preferably at least 46%, preferably at least 48, and most preferably at least 50% (w/w dry matter).

In a preferred embodiment of the third aspect, the primary food stream material has a fibre content of 4% (w/w dry matter) or less, preferably 3% (w/w dry matter) or less, preferably 2% (w/w dry matter) or less, and most preferably 1% (w/w dry matter) or less.

In a preferred embodiment of the third aspect, the second plant-based hydrolysate prepared from primary food stream material has been prepared from soy bean, fava bean, wheat, rice, pea, potato, or cotton seed.

In a preferred embodiment of the third aspect, the second plant-based hydrolysate obtained from primary food stream is present in a concentration in the range from 0.1% to 50% (w/w).

In a preferred embodiment of the third aspect, the second plant-based hydrolysate obtained from primary food stream was prepared by applying the method of the first aspect. This means that a plant-based material obtained from primary food stream, as defined above, is provided and that then steps (b) to (g) of the method of the first aspect are carried out.

In some embodiments of the invention, the composition according to the third aspect is used as a replacement of cell culture medium. An aqueous solution of the composition according to the third aspect can be prepared, thereby obtaining a plant-based medium. This plant-based medium can be used to partially replace a standard cell culture medium. For example, from about 100 mL to about 500 mL (preferably from about 150 mL to about 450 mL, preferably from about 200 mL to about 400 mL, more preferably from about 250 mL to about 350 mL, even more preferably about 300 mL) are removed from 1 L of a standard cell culture medium and the same volume of the plant-based medium as the volume that was removed is added to the remaining volume of standard cell culture medium as a replacement. For example, 300 mL are removed from 1 L of a standard cell culture medium and 300 mL of the plant-based medium are added to the remaining 700 mL as a replacement.

In other embodiments of the invention, the composition according to the third aspect is used as the starting material for the preparation of a cell culture medium. Further components (salts, buffer, energy sources (e.g. glucose), trace minerals, essential amino acids, growth factors) can then be added. Osmolality and pH can be adjusted. A person skilled in the art will be well-aware which further components, which pH and which osmolality will be suitable to promote growth of eukaryotic cells.

In a fourth aspect the present invention is directed to a method for cultivating cells, comprising the steps:
(a) Dissolving the composition according to the third aspect in a sterile aqueous solvent, thereby preparing a solution of a plant hydrolysate;
(b) Adding salts, buffer, energy sources (e.g. glucose), trace minerals, essential amino acids, and/or growth factors to the solution of step (a), thereby preparing a growth medium;
(c) Inoculating the growth medium of step (b) with appropriate cells, wherein the appropriate cells are selected from the group consisting of myosatellite cells, mesenchymal stromal cells, fibroblast cells, muscle cells, stem cells, and induced pluripotent stem cells (iPSCs); and
(d) Culturing the inoculated growth medium of step (c) at an appropriate temperature for an appropriate time period, thereby cultivating the cells.

In a preferred embodiment of the fourth aspect, the sterile aqueous solvent is selected from the group consisting of water, phosphate buffer, and a solution of sodium chloride.

In a preferred embodiment of the fourth aspect, the appropriate cells are bovine cells, porcine cells or chicken cells.

In embodiments, in which the stem cells are human embryonic stem cells, it is obligatory that these human embryonic stem cells were prepared by a process that does not require the destruction of the human embryo.

A person skilled in the art of producing cultured meat will be well aware which temperature ranges and which time-periods are required for obtaining cultured meat, taking into consideration the type of cells and the amount of cells used for inoculation.

In a first aspect the present invention is directed to a method for the production of a plant-based hydrolysate suitable for cell culture of eukaryotic cells, said plant-based protein hydrolysate being obtained from secondary food stream, comprising the steps:
(a) providing a plant-based material obtained from secondary food stream, wherein the plant-based material obtained from secondary food stream is obtained from rice bran, wherein said plant-based material obtained from secondary food stream has a protein content ranging from 5% to 25% (w/w dry matter) and has a fibre content ranging from 1% to 30% (w/w dry matter);
(b) adding a concentrated solution of HCl to the plant-based material, wherein the concentration of HCl is in the range from 0.2 M to 1 M, thereby preparing a mixture of acid and material;
(c) incubating the mixture of step (b) at a temperature in the range between 70°C to 90°C for a time period of between 15 hours and 36 hours, thereby preparing an acid-hydrolysate;
(d) adding an alkaline solution to the acid-hydrolysate of step (c) in an amount sufficient to neutralize the acid-hydrolysate;
(e) centrifuging the neutralized acid-hydrolysate of step (d) to precipitate insoluble material;
(f) recovering the supernatant of the centrifugation of step (e); and
(g) filtering the supernatant recovered in step (f) through a sterile filter or a combination of sterile filters, thereby obtaining the plant-based hydrolysate suitable for cell culture of eukaryotic cells.

In a preferred embodiment of the first aspect, the plant-based material obtained from secondary food stream has a protein content ranging from 6% to 25%, preferably from 7% to 25%, preferably from 8% to 25%, preferably from 9% to 25%, preferably from 10% to 25%, preferably from 11% to 25%, preferably from 12% to 25%, preferably from 13% to 25%, preferably from 14% to 25%, and most preferably from 15% to 25% (w/w dry matter).

In a preferred embodiment of the first aspect, the plant-based material obtained from secondary food stream has a fibre content ranging from 2% to 30%, preferably from 3% to 30%, preferably from 4% to 30%, preferably from 5% to 30%, and most preferably from 6% to 25% (w/w dry matter).

In all embodiments of the first aspect, the plant-based material obtained from secondary food stream is or is obtained from rice bran.

Further disclosed herein but not claimed are methods, in which the secondary food stream material is or is prepared from wheat bran, rye bran, maize bran, spelt bran, or brewer's spent grain.

In a preferred embodiment of the first aspect, the plant-based material obtained from secondary food stream is or is prepared from
- rice bran having a protein content in the range from 5% to 20% (w/w dry matter) and a fibre content in the range from 2% to 30% (w/w dry matter).

Further disclosed herein but not claimed are methods, in which the secondary food stream material is or is prepared from
- wheat bran having a protein content in the range from about 12% to about 24% (w/w dry matter) and a fibre content in the range from about 5% to about 15% (w/w dry matter); or
- rye bran having a protein content in the range from about 12% to about 20% (w/w dry matter) and a fibre content in the range from about 4% to about 30% (w/w dry matter); or
- maize bran having a protein content in the range from about 5% to about 20% (w/w dry matter) and a fibre content in the range from about 4% to about 25% (w/w dry matter); or
- spelt bran having a protein content in the range from about 12% to about 24% (w/w dry matter) and a fibre content in the range from about 5% to about 16% (w/w dry matter); or
- brewer's spent grain having a protein content in the range from about 16% to about 35% (w/w dry matter) and a fibre content in the range from about 10% to about 25% (w/w dry matter).

In a preferred embodiment of the first aspect, the concentration of HCl is in the range from 0.3 M to 1 M, more preferably in the range from 0.4 M to about 0.75 M, and most preferably at about 0.5 M.

In a preferred embodiment of the first aspect, the incubation of step (c) is carried out at a temperature in the range between 71°C and 89°C, preferably at a temperature in the range between 72°C and 88°C, preferably at a temperature in the range between 73°C and 87°C, preferably at a temperature in the range between 74°C and 86°C, preferably at a temperature in the range between 75°C and 85°C, preferably at a temperature in the range between 76°C and 84°C, preferably at a temperature in the range between 77°C and 83°C, preferably at a temperature in the range between 78°C and 82°C, preferably at a temperature in the range between 79°C and 81°C, and most preferably at about 80°C.

In a preferred embodiment of the first aspect, step (c) is carried out for a time period between 18 hours and 30 hours, and most preferably for about 24 hours.

In a preferred embodiment of the first aspect, step (e) is carried out for about 5 min to about 20 min at about 4000 to 6000 x g, preferably for about 10 min to 15 min at about 4500 to about 5500 x g; most preferably for about 10 min at about 5250 x g.

In a preferred embodiment of the first aspect, the sterile filter is a filter with a pore size of 50 µm or less, preferably 20 µm or less, more preferably 10 µm or less, and even more preferably, 0.2 µm or less.

In preferred embodiments of the first aspect, several filtration steps with increasingly smaller pore sizes are used; e.g. a first filtration step with a filter having a pore size of 50 µm or less, a second filtration step with a filter having a pore size of 20 µm or less, a third filtration step with a filter having a pore size of 10 µm or less, and a fourth filtration step with a filter having a pore size of 0.2 µm or less.

As stated above, the method of the first aspect and its preferred embodiments are also suitable for producing a plant-based hydrolysate obtained from primary food stream when using a plant-based material obtained from primary food stream as starting material.

In a second aspect the present invention is directed to a plant-based hydrolysate obtainable by the method of the first aspect.

In a preferred embodiment of the second aspect, the plant-based hydrolysate has been obtained by the method of the first aspect.

In a fifth aspect the present invention is directed to a method for the production of the composition of the third aspect, comprising the step(s):
- mixing a plant-based hydrolysate obtainable by the method of the first aspect with a food grade premix and optionally with a plant-based hydrolysate prepared from primary food stream material by acid hydrolysis.

In a preferred embodiment of the fifth aspect, the plant-based hydrolysate prepared from secondary food stream has been obtained by the method of the first aspect.

In preferred embodiments of the fifth aspect, the secondary food stream material is as defined in the embodiments of the third aspect.

In a preferred embodiment of the fifth aspect, the food grade premix is defined as in the embodiments of the third aspect of the invention.

In preferred embodiments of the fifth aspect, the primary food stream material is as defined in the embodiments of the third aspect.

The following figures and examples are merely illustrative of the present invention and should not be construed to limit the scope of the invention as indicated by the appended claims in any way.

### BRIEF DESCRIPTION OF THE FIGURES

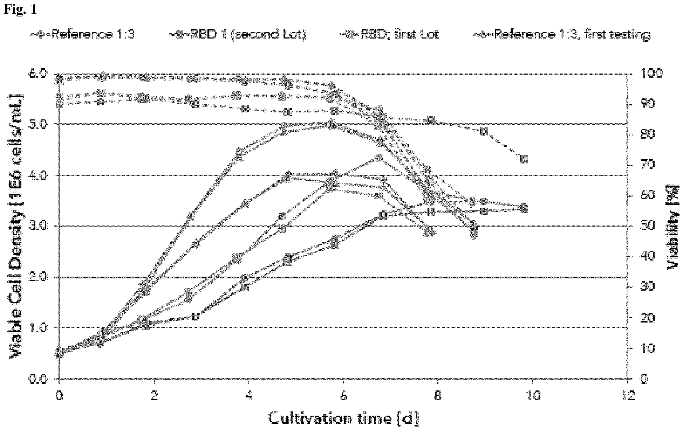

Fig. 1 shows a cell culture experiment with CHO cells. CHO cells were grown in reference medium or in media, in which components of the reference medium were replaced by a hydrolysate of defatted rice bran (RBD) (20% protein content). Two different lots of RBD hydrolysate were tested. At each day of cultivation, viable cell density (in 10⁶ cells/mL) and viability of cells were recorded for each experiment.

### EXAMPLES

### 1. Acid Hydrolysis of Plant Material

The plant material (either from primary food stream or from secondary food stream) was hydrolysed using the following protocol:
- 5 g of plant material was added to an empty 100 ml flask.
- 50 mL of 0.5 M HCl was added to the plant material.
- The mixture of plant material and HCl was mixed and incubated at 80°C for about 24 hours.
- Different solutions of NaOH were slowly and consecutively added until a pH of 7.0 was reached: First a 5 M NaOH solution was added until a pH of about 5 was reached; then a 1 M NaOH solution was added until a pH of about 6 was reached; finally, a 0.1 M NaOH solution was added until pH 7.0 was reached.
- The neutralized mixture was transferred to a new 100 ml flask.
- Distilled water was added up to a total volume of 100 mL.
- The flask was closed by a lid and was shaken. The temperature was adjusted to 20°C.
- The mixture was transferred to centrifugation tubes (2 tubes with a size of 50 mL each). The tubes were centrifuged at 4000 rpm for 10 min in an Allegra X-15R centrifuge (Beckman Coulter); corresponding to 5250 x g.
- The supernatant was collected and filtered through a 10 µm Teflon filter, thereby obtaining a sterile solution of plant hydrolysate.

Plant hydrolysates were prepared from the following starting materials (protein content is indicated in brackets): rye bran (17.1%), wheat bran (14.3%), fava bean (66%), spelt bran (19.7%), legria (21.2%), stabilized rice bran (15.1%), and defatted rice bran (19.0%).

The above-described protocol was also carried out in a larger scale, in which all materials were used in 50-fold amount:
- 250 g of plant material was added to an empty 5 L flask.
- 2.5 L of 0.5 M HCl was added to the plant material.
- The mixture of plant material and HCl was mixed and incubated at 80°C for about 24 hours.
- Different solutions of NaOH were slowly and consecutively added until a pH of 7.0 was reached: First a 5 M NaOH solution was added until a pH of about 5 was reached; then a 1 M NaOH solution was added until a pH of about 6 was reached; finally, a 0.1 M NaOH solution was added until pH 7.0 was reached.
- The neutralized mixture was transferred to a new 5 L flask.
- Distilled water was added up to a total volume of 5 L.
- The flask was closed by a lid and was shaken. The temperature was adjusted to 20°C.
- The mixture was transferred to centrifugation tubes (8 tubes with a size of 750 mL each). The tubes were centrifuged for 10 min at 5250 x g.
- The supernatant was collected and filtered through a 10 µm Teflon filter, thereby obtaining a sterile solution of plant hydrolysate.

### 2. Cell culture experiments

### Materials:

| | |
|---|---|
| CHO-K1 cells | Chinese hamster ovary cell line, subclone K1 |
| Reference medium | Liquid SBF medium, obtainable from Xell AG (Bielefeld, Germany) upon request under the product number 2052-0001 |

Cell culture experiments were carried out by Xell AG in their fermenters in Bielefeld as contract work. CHO-K1 cells were grown in reference medium or in a medium containing a replacement with a hydrolysate of defatted rice bran (RBD) (20% protein content). The hydrolysates of defatted rice bran had been prepared according to the acid hydrolysis protocol described in Example 1 above. Two different lots of RBD hydrolysate were tested.

Growth curves for the reference medium 1:3 and for the two experiments with two separate lots of RBD hydrolysate are shown in Fig. 1. Reference medium 1:3 means that 300 mL per 1 L of reference medium were replaced by distilled water. RBD and RBD1 both refer to a medium composition, in which 300 mL of defatted rice bran medium were added to 700 mL reference medium. Glucose concentration in all media variants was adjusted to either 4.5 g/L (for RBD and RBD1) or 6.2 g/L (for Reference 1:3), depending of glucose concentration of the food grade materials used. Glutamine was added to a final concentration of 6 mM. Cells were cultivated in 125 mL plain shake flasks with a working volume of 50 mL (in growth curves) in a 37°C incubator with an atmosphere of 5 % CO₂.

As can be seen from the growth curves, media containing a replacement of RBD hydrolysate allow growth of CHO cells. No inhibitory effect and no cytotoxicity were observed.

However, the maximum cell density was lower when using a medium containing a replacement of rice bran hydrolysate (about 4 × 10⁶ cells/mL for the first lot; about 3.5 x 10⁶ cells/mL for the second lot) as compared to the reference medium (5 × 10⁶ cells/mL). In addition, maximum cell density was reached only after longer cultivation times (at day 7 for the first lot; at day 8 for the second lot) as compared to the reference medium (at day 6).

In summary, the above results provide a proof of principle that a hydrolysate from rice bran (i.e. from a secondary food stream material) can be used as a supplement in cell culture media and supports the growth of eukaryotic cells.

## Claims

1. A method for the production of a plant-based protein hydrolysate suitable for cell culture of eukaryotic cells, said plant-based protein hydrolysate being obtained from secondary food stream, the method comprising the steps:
(a) providing a plant-based material obtained from secondary food stream, wherein the plant-based material obtained from secondary food stream is obtained from rice bran, wherein said plant-based material obtained from secondary food stream has a protein content ranging from 5% to 25% (w/w dry matter) and has a fibre content ranging from 1% to 30% (w/w dry matter);
(b) adding a concentrated solution of HCl to the plant-based material, wherein the concentration of HCl is in the range from 0.2 M to 1 M, thereby preparing a mixture of acid and material;
(c) incubating the mixture of step (b) at a temperature in the range between 70°C and 90°C for a time period of between 15 hours and 36 hours, thereby preparing an acid-hydrolysate;
(d) adding an alkaline solution to the acid-hydrolysate of step (c) in an amount sufficient to neutralize the acid-hydrolysate;
(e) centrifuging the neutralized acid-hydrolysate of step (d) to precipitate insoluble material;
(f) recovering the supernatant of the centrifugation of step (e); and
(g) filtering the supernatant recovered in step (f) through a sterile filter or a combination of sterile filters, thereby obtaining the plant-based protein hydrolysate suitable for cell culture of eukaryotic cells.

2. The method according to claim 1, wherein the concentration of HCl is in the range from 0.3 M to 0.75 M.

3. The method according to any one of claims 1 to 2, wherein step (c) is carried out
- at a temperature between 75°C and 85°C, preferably at 80°C; and/or
- for a time period between 18 hours and 30 hours, preferably for 24 hours.

4. The method according to any one of claims 1 to 3, wherein step (e) is carried out for 5 to 20 minutes at 4000 to 6000 x g.

5. The method according to any one of claims 1 to 4, wherein the sterile filter is a filter with a pore size of 50 µm or less.

6. A plant-based protein hydrolysate (obtained from using secondary food streams), obtainable by the method of any one of claims 1 to 5.

7. A composition suitable for use as medium component supporting the growth of eukaryotic cells, the composition comprising:
(i) a first plant-based protein hydrolysate obtainable by the method of any one of claims 1 to 5 and
(ii) a food grade premix comprising one or more minerals, one or more vitamins, one or more amino acids, and/or one or more salts;
wherein said composition is devoid of animal proteins.

8. The composition according to claim 7, wherein the first plant-based protein hydrolysate is present in the composition in a concentration in the range from 0.1% to 50% (w/w).

9. The composition according to any one of claims 7 to 8, wherein
- the one or more minerals in the food grade pre-mix are selected from the group consisting of Mg²⁺, Ca²⁺, Fe²⁺, Fe³⁺, Cu²⁺, Zn²⁺, and Mn²⁺;
- the one or more vitamins in the food grade pre-mix are selected from the group consisting of ascorbic acid, biotin, choline, D-calcium pantothenate, folic acid, niacinamide, pyridoxine, pyridoxal, riboflavin, thiamine, vitamin B12, and i-inositol;
- the one or more amino acids in the food grade pre-mix are selected from the group consisting of glycine, L-alanine, L-arginine, L-asparagine, L-aspartic acid, L-cysteine, L-cystine, L-glutamic acid, L-glutamine, L-histidine, L- isoleucine, L-leucine, L-lysine, L- methionine, L-phenylalanine, L-proline, L-serine, L- threonine, L-tryptophan, L-tyrosine, and L-valine; and/or
- the one or more salts in the food grade pre-mix are selected from the group consisting of NaCl, KCl, Na₂HPO₄, NaH₂PO₄, and NaHCO₃.

10. The composition according to any one of claims 7 to 9, wherein the food grade premix is present in the composition in a concentration in the range from 10% to 99.9% (w/w).

11. The composition according to any one of claims 7 to 10, further comprising:
(iii) a second plant-based protein hydrolysate prepared from primary food stream material by acid hydrolysis, wherein said primary food stream material has a protein content of at least 30% (w/w dry matter) protein and a fibre content of 5% (w/w dry matter) or less,
wherein preferably the primary food stream material has been prepared from soy bean, fava bean, wheat, rice, pea, potato, or cotton seed.

12. The composition according to claim 11, wherein the second plant-based protein hydrolysate obtained from primary food stream material is present in a concentration in the range from 0.1% to 50% (w/w).

13. A method for cultivating cells, comprising the steps:
(a) dissolving the composition according to any one of claims 7 to 12 in a sterile aqueous solvent, thereby preparing a solution of a plant hydrolysate;
(b) adding salts, buffer, energy sources (e.g. glucose), trace minerals, essential amino acids, and/or growth factors to the solution of step (a), thereby preparing a growth medium;
(c) inoculating the growth medium of step (b) with appropriate cells, wherein the appropriate cells are selected from the group consisting of myosatellite cells, mesenchymal stromal cells, fibroblast cells, muscle cells, stem cells, and induced pluripotent stem cells (iPSCs); and
(d) culturing the inoculated growth medium of step (c) at an appropriate temperature for an appropriate time period, thereby cultivating the cells.

14. A method for the production of the composition of any one of claims 7 to 12, comprising the step(s):
- mixing a plant-based protein hydrolysate obtainable by the method of any one of claims 1 to 5 with a food grade premix and optionally with a plant-based protein hydrolysate prepared from primary food stream material by acid hydrolysis.

## Patentansprüche

1. Verfahren zur Herstellung eines pflanzlichen Proteinhydrolysats, das für eine Zellkultur eukaryotischer Zellen geeignet ist, wobei das pflanzliche Proteinhydrolysat aus einem sekundären Nahrungsmittelstrom erhalten wird, wobei das Verfahren die folgenden Schritte umfasst:
(a) Bereitstellen eines pflanzlichen Materials, das aus einem sekundären Nahrungsmittelstrom erhalten wird, wobei das aus dem sekundären Nahrungsmittelstrom erhaltene pflanzliche Material aus Reiskleie erhalten wird, wobei das aus dem sekundären Nahrungsmittelstrom erhaltene pflanzliche Material einen Proteingehalt im Bereich von 5 Gew% bis 25 Gew% (Trockenmasse) aufweist und einen Ballaststoffgehalt im Bereich von 1 Gew% bis 30 Gew% (Trockenmasse) aufweist;
(b) Zugeben einer konzentrierten Lösung von HCl zu dem pflanzlichen Material, wobei die Konzentration von HCl im Bereich von 0,2 M bis 1 M liegt, um dadurch ein Gemisch aus Säure und Material herzustellen;
(c) Inkubieren des Gemischs von Schritt (b) bei einer Temperatur im Bereich zwischen 70 °C und 90 °C für eine Zeitdauer zwischen 15 Stunden und 36 Stunden, um dadurch ein Säurehydrolysat herzustellen;
(d) Zugeben einer alkalischen Lösung zu dem Säurehydrolysat von Schritt (c) in einer Menge, die ausreicht, um das Säurehydrolysat zu neutralisieren;
(e) Zentrifugieren des neutralisierten Säurehydrolysats von Schritt (d), um unlösliches Material auszufällen;
(f) Gewinnen des Überstands der Zentrifugation von Schritt (e); und
(g) Filtrieren des in Schritt (f) gewonnenen Überstands durch einen Sterilfilter oder eine Kombination von Sterilfiltern, um dadurch das für die Zellkultur eukaryotischer Zellen geeignete pflanzliche Proteinhydrolysat zu erhalten.

2. Verfahren nach Anspruch 1, wobei die Konzentration von HCl im Bereich von 0,3 M bis 0,75 M liegt.

3. Verfahren nach einem der Ansprüche 1 bis 2, wobei Schritt (c) ausgeführt wird
- bei einer Temperatur zwischen 75 °C und 85 °C, vorzugsweise bei 80°C; und/oder
- für eine Zeitdauer zwischen 18 Stunden und 30 Stunden, vorzugsweise 24 Stunden.

4. Verfahren nach einem der Ansprüche 1 bis 3, wobei Schritt (e) für 5 bis 20 Minuten bei 4000 bis 6000 x g ausgeführt wird.

5. Verfahren nach einem der Ansprüche 1 bis 4, wobei der Sterilfilter ein Filter mit einer Porengröße von 50 µm oder weniger ist.

6. Pflanzliches Proteinhydrolysat (erhalten unter Verwendung sekundärer Nahrungsmittelströme), erhältlich durch das Verfahren nach einem der Ansprüche 1 bis 5.

7. Zusammensetzung, die zur Verwendung als Mediumkomponente geeignet ist, die das Wachstum eukaryotischer Zellen unterstützt, wobei die Zusammensetzung Folgendes umfasst:
(i) ein erstes pflanzliches Proteinhydrolysat, das durch das Verfahren nach einem der Ansprüche 1 bis 5 erhältlich ist, und
(ii) eine Vormischung in Lebensmittelqualität, die ein oder mehrere Mineralien, ein oder mehrere Vitamine, eine oder mehrere Aminosäuren und/oder ein oder mehrere Salze umfasst;
wobei die Zusammensetzung frei von tierischen Proteinen ist.

8. Zusammensetzung nach Anspruch 7, wobei das erste pflanzliche Proteinhydrolysat in der Zusammensetzung in einer Konzentration im Bereich von 0,1 Gew% bis 50 Gew% vorhanden ist.

9. Zusammensetzung nach einem der Ansprüche 7 bis 8, wobei
- das eine oder die mehreren Minerale in der Vormischung in Lebensmittelqualität ausgewählt sind aus der Gruppe bestehend aus Mg²⁺, Ca²⁺, Fe²⁺, Fe³⁺, Cu²⁺, Zn²⁺, und Mn²⁺;
- das eine oder die mehreren Vitamine in der Vormischung in Lebensmittelqualität ausgewählt sind aus der Gruppe bestehend aus Ascorbinsäure, Biotin, Cholin, D-Calciumpantothenat, Folsäure, Niacinamid, Pyridoxin, Pyridoxal, Riboflavin, Thiamin, Vitamin B12 und i-Inositol;
- die eine oder die mehreren Aminosäuren in der Vormischung in Lebensmittelqualität ausgewählt sind aus der Gruppe bestehend aus Glycin, L-Alanin, L-Arginin, L-Asparagin, L-Asparaginsäure, L-Cystein, L-Cystin, L-Glutaminsäure, L-Glutamin, L-Histidin, L-Isoleucin, L-Leucin, L-Lysin, L-Methionin, L-Phenylalanin, L-Prolin, L-Serin, L-Threonin, L-Tryptophan, L-Tyrosin und L-Valin; und/oder
- das eine oder die mehreren Salze in der Vormischung in Lebensmittelqualität ausgewählt sind aus der Gruppe bestehend aus NaCl, KCl, Na₂HPO₄, NaH₂PO₄ und NaHCO₃.

10. Zusammensetzung nach einem der Ansprüche 7 bis 9, wobei das erste pflanzliche Proteinhydrolysat in der Zusammensetzung in einer Konzentration im Bereich von 10 Gew% bis 99,9 Gew% vorhanden ist.

11. Zusammensetzung nach einem der Ansprüche 7 bis 10, ferner umfassend:
(iii) ein zweites pflanzliches Proteinhydrolysat, das aus Material eines primärem Nahrungsmittelstroms durch Säurehydrolyse hergestellt ist, wobei das Material des primären Nahrungsmittelstroms einen Proteingehalt von mindestens 30 Gew% (Trockenmasse) und einen Ballaststoffgehalt von 5 Gew% (Trockenmasse) oder weniger aufweist,
wobei das Material des primären Nahrungsmittelmaterials vorzugsweise aus Sojabohnen, Favabohnen, Weizen, Reis, Erbsen, Kartoffeln oder Baumwollsamen hergestellt ist.

12. Zusammensetzung nach Anspruch 11, wobei das zweite pflanzliche Proteinhydrolysat, das aus Material eines primären Nahrungsmittelstroms erhalten wird, in einer Konzentration im Bereich von 0,1 Gew% bis 50 Gew% vorhanden ist.

13. Verfahren zur Kultivierung von Zellen, umfassend die folgenden Schritte:
(a) Lösen der Zusammensetzung nach einem der Ansprüche 7 bis 12 in einer sterilen wässrigen Lösung, um dadurch eine Lösung eines pflanzlichen Hydrolysats herzustellen;
(b) Zugeben von Salzen, Puffern, Energiequellen (z. B. Glukose), Spurenelementen, essenziellen Aminosäuren und/oder Wachstumsfaktoren zu der Lösung von Schritt (a), um dadurch ein Wachstumsmedium herzustellen;
(c) Beimpfen des Wachstumsmediums von Schritt (b) mit geeigneten Zellen, wobei die geeigneten Zellen ausgewählt werden aus der Gruppe bestehend aus Myosatellitenzellen, mesenchymalen Stromazellen, Fibroblastenzellen, Muskelzellen, Stammzellen und induzierten pluripotenten Stammzellen (iPSCs); und
(d) Kultivieren des beimpften Wachstumsmediums von Schritt (c) bei einer geeigneten Temperatur für eine geeignete Zeitdauer, um dadurch die Zellen zu kultivieren.

14. Verfahren zur Herstellung der Zusammensetzung nach einem der vorhergehenden Ansprüche 7 bis 12, umfassend den/die folgenden Schritt(e):
- Mischen eines pflanzlichen Proteinhydrolysats, das durch das Verfahren nach einem der Ansprüche 1 bis 5 erhältlich ist, mit einer Vormischung in Lebensmittelqualität und gegebenenfalls mit einem pflanzlichen Proteinhydrolysat, das durch Säurehydrolyse aus Material eines primären Nahrungsmittelstroms hergestellt wurde.

## Revendications

1. Procédé de production d'un hydrolysat de protéines d'origine végétale adapté à la culture cellulaire de cellules eucaryotes, ledit hydrolysat de protéines d'origine végétale étant obtenu à partir d'un flux alimentaire secondaire, le procédé comprenant les étapes:
(a) la fourniture d'une matière d'origine végétale obtenue à partir d'un flux alimentaire secondaire, dans lequel la matière d'origine végétale obtenue à partir d'un flux alimentaire secondaire est obtenue à partir de son de riz, dans lequel ladite matière d'origine végétale obtenue à partir d'un flux alimentaire secondaire présente une teneur en protéines allant de 5% à 25% (p/p de matière sèche) et présente une teneur en fibres allant de 1% à 30% (p/p de matière sèche) ;
(b) l'ajout d'une solution concentrée de HCl à la matière d'origine végétale, dans lequel la concentration de HCl se trouve dans la plage allant de 0,2 M à 1 M, préparant ainsi un mélange d'acide et de matière ;
(c) l'incubation du mélange de l'étape (b) à une température dans la plage comprise entre 70°C et 90°C pendant une durée comprise entre 15 heures et 36 heures, préparant ainsi un hydrolysat acide ;
(d) l'ajout d'une solution alcaline à l'hydrolysat acide de l'étape (c) en une quantité suffisante pour neutraliser l'hydrolysat acide ;
(e) la centrifugation de l'hydrolysat acide neutralisé de l'étape (d) pour précipiter la matière insoluble ;
(f) la récupération du surnageant de la centrifugation de l'étape (e) ; et
(g) la filtration du surnageant récupéré à l'étape (f) à travers un filtre stérile ou une combinaison de filtres stériles, obtenant ainsi l'hydrolysat de protéines d'origine végétale adapté à la culture cellulaire de cellules eucaryotes.

2. Procédé selon la revendication 1, dans lequel la concentration de HCl se trouve dans la plage allant de 0,3 M à 0,75 M.

3. Procédé selon l'une quelconque des revendications 1 et 2, dans lequel l'étape (c) est réalisée
- à une température comprise entre 75°C et 85°C, de préférence à 80°C ; et/ou
- pendant une durée comprise entre 18 heures et 30 heures, de préférence pendant 24 heures.

4. Procédé selon l'une quelconque des revendications 1 à 3, dans lequel l'étape (e) est réalisée pendant 5 à 20 minutes entre 4000 et 6000 x g.

5. Procédé selon l'une quelconque des revendications 1 à 4, dans lequel le filtre stérile est un filtre avec une taille de pores inférieure ou égale à 50 µm.

6. Hydrolysat de protéines d'origine végétale (obtenu en utilisant des flux alimentaires secondaires), pouvant être obtenu par le procédé selon l'une quelconque des revendications 1 à 5.

7. Composition appropriée pour être utilisée comme composant de milieu favorisant la croissance de cellules eucaryotes, la composition comprenant :
(i) un premier hydrolysat de protéines d'origine végétale pouvant être obtenu par le procédé selon l'une quelconque des revendications 1 à 5 et
(ii) un prémélange de qualité alimentaire comprenant un ou plusieurs minéraux, une ou plusieurs vitamines, un ou plusieurs acides aminés et/ou un ou plusieurs sels ;
dans laquelle ladite composition est dépourvue de protéines animales.

8. Composition selon la revendication 7, dans laquelle le premier hydrolysat de protéines d'origine végétale est présent dans la composition à une concentration dans la plage allant de 0,1% à 50% (p/p).

9. Composition selon l'une quelconque des revendications 7 et 8, dans laquelle
- le ou les plusieurs minéraux dans le prémélange de qualité alimentaire sont choisis dans le groupe constitué de Mg²⁺, Ca²⁺, Fe²⁺, Fe³⁺, Cu²⁺, Zn²⁺ et Mn²⁺ ;
- la ou les plusieurs vitamines dans le prémélange de qualité alimentaire sont choisies dans le groupe constitué par l'acide ascorbique, la biotine, la choline, le D-pantothénate de calcium, l'acide folique, le niacinamide, la pyridoxine, le pyridoxal, la riboflavine, la thiamine, la vitamine B12 et l'i-inositol ;
- le ou les plusieurs acides aminés dans le prémélange de qualité alimentaire sont choisis dans le groupe constitué par la glycine, la L-alanine, la L-arginine, la L-asparagine, l'acide L-aspartique, la L-cystéine, la L-cystine, l'acide L-glutamique, la L-glutamine, la L-histidine, la L-isoleucine, la L-leucine, la L-lysine, la L-méthionine, la L-phénylalanine, la L-proline, la L-sérine, la L-thréonine, le L-tryptophane, la L-tyrosine et la L-valine ; et/ou
- le ou les plusieurs sels dans le prémélange de qualité alimentaire sont choisis dans le groupe constitué de NaCl, KCl, Na₂HPO₄, NaH₂PO₄, et NaHCOa.

10. Composition selon l'une quelconque des revendications 7 à 9, dans laquelle le prémélange de qualité alimentaire est présent dans la composition à une concentration dans la plage allant de 10% à 99,9% (p/p).

11. Composition selon l'une quelconque des revendications 7 à 10, comprenant en outre :
(iii) un second hydrolysat de protéines d'origine végétale préparé à partir de matière de flux alimentaire primaire par hydrolyse acide, dans laquelle ladite matière de flux alimentaire primaire présente une teneur en protéines d'au moins 30% (p/p de matière sèche) et une teneur en fibres inférieure ou égale à 5% (p/p de matière sèche),
dans laquelle de préférence la matière de flux alimentaire primaire a été préparée à partir de soja, de fève, de blé, de riz, de pois, de pomme de terre ou de graine de coton.

12. Composition selon la revendication 11, dans laquelle le second hydrolysat de protéines d'origine végétale obtenu à partir de matière de flux alimentaire primaire est présent à une concentration dans la plage allant de 0,1% à 50% (p/p).

13. Procédé de culture de cellules, comprenant les étapes :
(a) la dissolution de la composition selon l'une quelconque des revendications 7 à 12 dans un solvant aqueux stérile, préparant ainsi une solution d'un hydrolysat végétal ;
(b) l'ajout de sels, d'un tampon, de sources d'énergie (par exemple du glucose), d'oligo-éléments, d'acides aminés essentiels et/ou des facteurs de croissance à la solution de l'étape (a), préparant ainsi un milieu de croissance ;
(c) l'inoculation du milieu de croissance de l'étape (b) avec des cellules appropriées, dans lequel les cellules appropriées sont choisies dans le groupe constitué de cellules myosatellites, de cellules stromales mésenchymateuses, de cellules fibroblastes, de cellules musculaires, de cellules souches et de cellules souches pluripotentes induites (iPSC) ; et
(d) la cultivation du milieu de croissance inoculé de l'étape (c) à une température appropriée pendant une durée appropriée, cultivant ainsi les cellules.

14. Procédé de production de la composition selon l'une quelconque des revendications 7 à 12, comprenant la ou les étape(s) :
- le mélange d'un hydrolysat de protéines d'origine végétale pouvant être obtenu par le procédé selon l'une quelconque des revendications 1 à 5 avec un prémélange de qualité alimentaire et éventuellement avec un hydrolysat de protéines d'origine végétale préparé à partir de matière de flux alimentaire primaire par hydrolyse acide.
